# EUROPEAN PATENT APPLICATION

(11) **EP 4 578 416 A2**
(43) Date of publication of application: **02.07.2025**
(21) Application number: 24222719.7
(22) Date of filing: 23.12.2024
(51) Int. Cl.: A61B 18/14

(54) **BASKET CATHETER AND ELECTRODE ASSEMBLIES CONFIGURED FOR IMPROVED TISSUE CONTACT**

(30) Priority: 27.12.2023 US 202363615210 P; 20.11.2024 US 202418953519
(71) Applicant: Biosense Webster (Israel) Ltd., 2066717 Yokneam (IL)
(72) Inventor: KATO, Robert, Irvine, 92618 (US); GOO, Audrey Claire, Irvine, 92618 (US); VU, Audrey, Irvine, 92618 (US); RAO, Anand, Irvine, 92618 (US)
(74) Representative: Carpmaels & Ransford LLP

(57) **Abstract**

The disclosed technology includes an end effector comprising a plurality of spines extending along a longitudinal axis to define a basket assembly, the plurality of spines configured to bow radially outward from the longitudinal axis to define a radius of curvature with respect to the longitudinal axis and to transition between an expanded configuration and a collapsed configuration. Each spine of the plurality of spines can include a section extending radially outward from the radius of curvature defined by a remainder of each spine. The end effector can further include at least one electrode disposed on the section for each spine of the plurality of spines.

## Description

### CROSS REFERENCE TO RELATED APPLICATIONS

This application claims the benefit of priority under 35 U.S.C. § 119 to prior filed U.S. Provisional Patent Application No. 63/615,210, filed December 27, 2023 (Attorney Docket No.: 253757.000194 (BIO6733USPSP1)), the entire contents of which is hereby incorporated by reference as if set forth in full herein.

### FIELD

The present invention relates generally to basket catheter assemblies and more particularly to basket catheter assemblies comprising spring-loaded electrodes disposed on the spines, the electrodes configured for improved tissue contact.

### BACKGROUND

Intravascular catheters are commonly used for mapping and ablation of myocardial tissue. Intravascular catheters typically include an end effector having one or more electrodes that are configured to receive electrical signals from the tissue for mapping and/or to deliver ablative energy to the tissue ablation. To ensure the electrodes are correctly positioned for mapping or ablation, some end effectors include position sensing such as electromagnetic position sensing or active current location (ACL) technology. This position sensing technology can also be used to detect the quality of contact between an ablation electrode and target tissue.

In some existing catheters, the catheter includes spines configured to bow radially outward to cause the electrodes to contact tissue. The spines are generally constructed with a uniform radius of curvature along the entirety or a majority of the spines. Because the radius of curvature of the spines is uniform, when the basket catheter contacts target tissue, additional portion of the spines on either side of the electrodes are also in contact with target tissue. When determining the positions of such electrodes, a user may not be able to acquire detailed enough information in regard to the force being applied to the electrodes separate from the spines. As will be appreciated, it is desirable to acquire accurate data in regard to the force being applied to the electrodes as well as the location of the individual electrodes. Thus, there is a need in the art for methods of improving the detectability of tissue contact of electrodes while also acquiring accurate force data. The technology disclosed herein addresses these and other issues.

### SUMMARY

There is provided, in accordance with an example of the present disclosure, an end effector for a medical device comprising a plurality of spines extending along a longitudinal axis to define a basket assembly. The plurality of spines can be configured to bow radially outward from the longitudinal axis to define a radius of curvature with respect to the longitudinal axis and to transition between an expanded configuration and a collapsed configuration. Each spine of the plurality of spines can include a section extending radially outward from the radius of curvature defined by a remainder of each spine. The end effector can further include at least one electrode disposed on the section for each spine of the plurality of spines.

The disclosed technology can include an electrode assembly comprising an electrode body extending along a longitudinal axis and defining a recess and an electrode cap disposed at least partially in the recess. The electrode assembly can further include a spring member disposed in the recess between the electrode body and the electrode cap, the spring member configured to cause the electrode cap to extend outwardly from the electrode body.

Additional features, functionalities, and applications of the disclosed technology are discussed in more detail herein.

### BRIEF DESCRIPTION OF THE DRAWINGS

FIG. 1 is a schematic pictorial illustration of a medical system, in accordance with an example of the disclosed technology;
FIG. 2A is a perspective view of a basket catheter in an expanded configuration, in accordance with an example of the disclosed technology;
FIG. 2B is a side view of the basket catheter of FIG. 2A in a collapsed configuration in a sheath, in accordance with an example of the disclosed technology;
FIG. 3 is a front view of a basket catheter comprising a position sensor and in an expanded configuration, in accordance with an example of the disclosed technology;
FIG. 4A is a front view of a basket catheter in an expanded configuration, in accordance with an example of the disclosed technology;
FIG. 4B is a top view of the basket catheter in FIG. 4A, in accordance with an example of the disclosed technology;
FIG. 5A is a top perspective view of an electrode, in accordance with an example of the disclosed technology;
FIG. 5B is a side view of the electrode in FIG. 5A, in accordance with an example of the disclosed technology;
FIG. 6A is a front perspective view of a basket catheter comprising position sensors disposed along the spines and in an expanded configuration, in accordance with an example of the disclosed technology;
FIG. 6B is a top perspective view of three position sensors to be disposed on a spine of the basket catheter in FIG. 6A, in accordance with an example of the disclosed technology;
FIG. 7 is a perspective view of a basket catheter comprising multiple electrode assemblies disposed along the spine, in accordance with an example of the disclosed technology;
FIG. 8A is a perspective view of an electrode assembly comprising an electrode cap, in accordance with an example of the disclosed technology;
FIG. 8B is a top view of the electrode assembly of FIG. 8A, in accordance with an example of the disclosed technology;
FIG. 8C is a side-front view of the electrode assembly of FIG. 8A, in accordance with an example of the disclosed technology;
FIG. 8D is a side-side view of the electrode assembly of FIG. 8A, in accordance with an example of the disclosed technology;
FIG. 9 is a cross-sectional view of the electrode assembly taken along line A-A illustrated in FIGS. 8B, in accordance with an example of the disclosed technology;
FIG. 10 is an exploded view of the electrode assembly illustrated in FIGS. 8A to 9, in accordance with an example of the disclosed technology;
FIG. 11A is a perspective view of a spring member comprising a strain gauge, in accordance with an example of the disclosed technology;
FIG. 11B is a top view of three strain gauges to be disposed on the spring member of FIG. 11A, in accordance with an example of the disclosed technology; and
FIG. 11C is a bottom perspective view an electrode body comprising electromagnetic coils, in accordance with an example of the disclosed technology.

### DETAILED DESCRIPTION

The disclosed technology includes a basket catheter that can be configured for ablation and/or mapping. As will become apparent throughout this disclosure, the spines of the basket catheter and the electrode configurations can be configured to help improve the tissue contact of the electrodes. The disclosed technology also includes improved methods of detecting a force applied to each electrode as well as the position of each electrode. In some examples, the spines can include biased sections that cause the electrodes disposed along the biased sections to contact tissue prior to the remainder of the spines contacting the tissue. This helps to ensure that the desired portions of the spines are contacting the target tissue first and that overall electrode surface contact is improved. In other examples, the spines can include location sensors and/or strain gauges disposed along the spines to acquire position and force data associated with the electrodes.

The disclosed technology also includes electrode assemblies that are configured to detect the contact force and the position of each individual electrode. For example, the electrode assemblies can each include a sensor to determine the force applied to the electrode assembly as well as the position and orientation of the electrode. That is, the sensor described herein allows a determination of the location of the electrode as well as the direction of a force being applied to the electrode. This is accomplished via the determination of a location of the magnetic coils (which gives the location of the electrode) and knowing the displacement of one of the magnetic coils relative to the other magnetic coil coupled to a spring (with a known spring constant) between the two magnetic coils allow for determining force and its vector.

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as reversable or irreversible electroporation (IRE), referred throughout this disclosure interchangeably as pulsed electric field (PEF) and pulsed field ablation (PFA), or thermal energy such as radiofrequency (RF) ablation or cryoablation. Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer or inner surface without departing from the scope of the present disclosure.

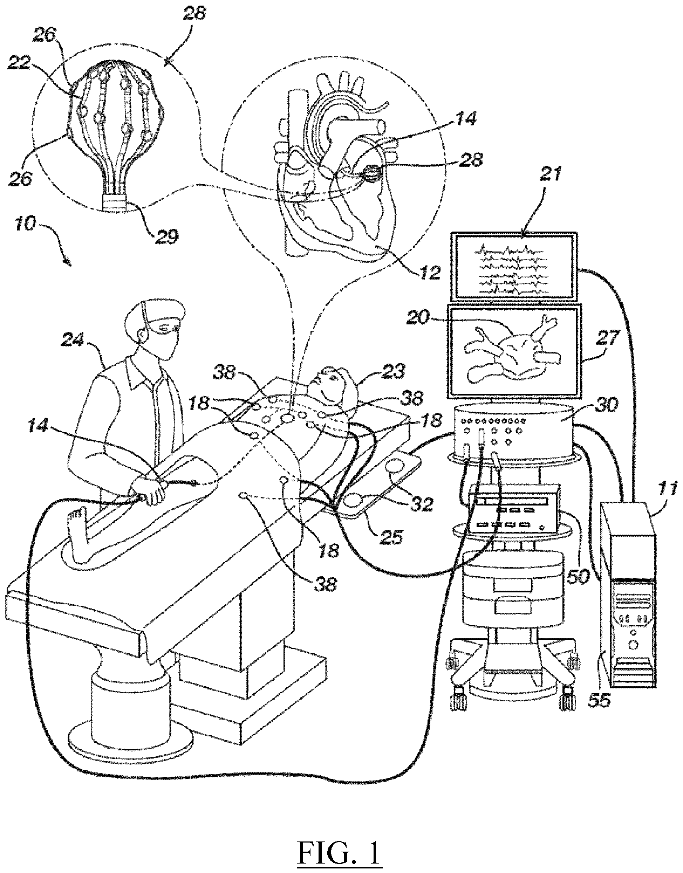

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. For sensing IEGM, Physician 24 brings a distal tip 28 of catheter 14 into contact with the heart wall for sensing a target site in heart 12. The end effector of the mapping catheter may include a basket catheter, a planar catheter, a focal catheter, a balloon catheter, etc. For ablation, physician 24 would similarly bring a distal end of an ablation catheter to a target site for ablating (as shown in inset of FIG. 1). Similarly, the end effector of the ablation catheter may include a basket catheter, a planar catheter, a focal catheter, a balloon catheter, etc.

Catheter 14 is an exemplary catheter that includes one and preferably multiple electrode assemblies 26 optionally distributed over a plurality of spines 22 at distal tip 28 and configured to deliver ablative energy to tissue. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

Magnetic based position sensor 29 may be operated together with a location pad 25 including a plurality of magnetic coils 32 configured to generate magnetic fields in a predefined working volume. Real time position of distal tip 28 of catheter 14 may be tracked based on magnetic fields generated with location pad 25 and sensed by magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, the entireties of each of which are incorporated herein by reference. Sensors that provide both contact force and position information via magnetic location technology are shown and described in detail in US Patent Nos. 8,437,832; 8,535,308; 8,784,413; and 8,357,152 the entireties of each of which are incorporated herein by reference and included in the Appendix filed herewith.

System 10 includes one or more electrode patches 38 positioned for skin contact on patient 23 to establish location reference for location pad 25 as well as impedance-based tracking of electrode assemblies 26. For impedance-based tracking, electrical current is directed toward electrode assemblies 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, the entireties of each of which are incorporated herein by reference.

A recorder 11 displays electrograms 21 captured with body surface ECG electrodes 18 and intracardiac electrograms (IEGM) captured with electrode assemblies 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618, USA.

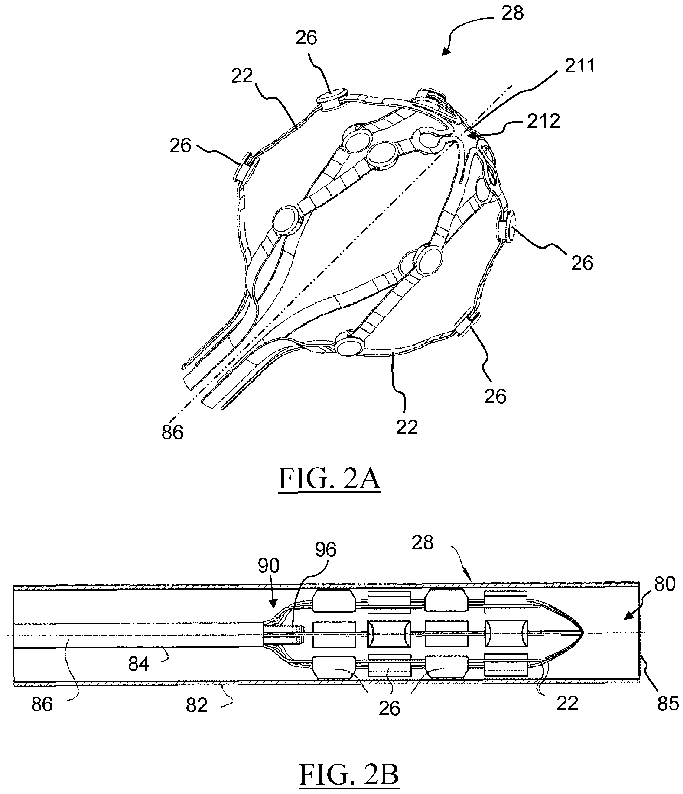

FIG. 2A is a schematic pictorial illustration showing a perspective view of a catheter 14 having an end effector 28 that is a basket assembly in an expanded form when unconstrained, such as by being advanced out of a tubular shaft lumen 80 at a distal end 85 of a tubular shaft 82 (as depicted in FIG. 2B). FIG. 2B shows the basket assembly in a collapsed form within tubular shaft 82. In the expanded form (FIG. 2A), the spines 22 bow radially outward along a longitudinal axis 86 and in the collapsed form (FIG. 2B) the spines are constrained generally along the longitudinal axis 86 of tubular shaft 82 by an inner wall of the tubular shaft 82.

As shown in FIG. 2A, basket assembly 28 (referred to herein interchangeably as "distal tip") includes a plurality of flexible spines 22 that are formed at the end of a flexible shaft 84 and are connected at both ends. During a medical procedure, physician 24 can deploy basket assembly 28 by extending flexible shaft 84 from tubular shaft 82 causing the basket assembly 28 to exit the tubular shaft 82 and transition to the expanded form. Spines 22 may have elliptical (e.g., circular) or rectangular (that may appear to be flat) cross-sections, and include a flexible, resilient material (e.g., a shape-memory alloy such as nickel-titanium, also known as Nitinol) forming a strut as will be described in greater detail herein.

In examples described herein, electrode assemblies 26 can be configured to deliver ablation energy (RF and/or IRE) to tissue in heart 12 or other parts of patient's 23 body. In addition to using electrode assemblies 26 to deliver ablation energy, the electrode assemblies can also be used to measure a physiological property such as local surface electrical potentials at respective locations on tissue in heart 12 (e.g., IEGM signals). The electrode assemblies 26 can be biased such that the larger surface area of the electrode body of the electrode assemblies 26 faces outwardly from the basket assembly 28 such that the electrode assemblies26 deliver a greater amount of electrical energy outwardly away from the basket assembly 28 (i.e., toward the heart 12 tissue) than inwardly toward the longitudinal axis 86 of the basket assembly 28. Basket assembly 28 can include a stem 96 that extends longitudinally from a distal end 90 of the flexible shaft 84 towards distal end 212 of basket assembly 28. The basket assembly 28 can include a central intersection 211 at a point where the spines 22 converge near the distal end 212.

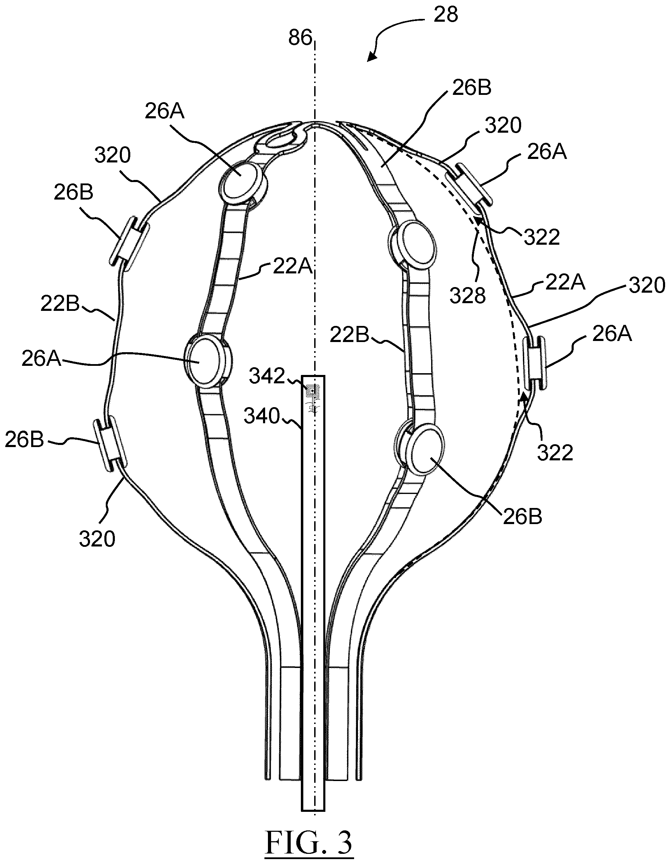

Turning now to FIG. 3 which illustrates an example end effector 28 in an expanded configuration. The end effector 28 can be configured to transition between the expanded configuration and a collapsed configuration. For example, the spines 22 can comprise nitinol and the nitinol can be heat-set to a predetermined shape (e.g., the deployed configuration) such that when the spines 22 are inserted into a blood pool at a predetermined temperature, the spines 22 will transition to the heat-set deployed configuration. In some examples, each spine 22 of the plurality of spines 22 can be covered in a layer of biocompatible insulative material. The end effector 28 can include a plurality of spines 22, each spine 22 of the plurality of spines 22 extending along the longitudinal axis 86 and configured to bow radially outward from the longitudinal axis 86 to define a radius of curvature 328 with respect to the longitudinal axis 86 and form a basket shape.

As shown in FIG. 3, each spine 22 of the plurality of spines 22 can further include a plurality of electrodes 26 disposed on each spine 22. The electrodes 26 can be disposed on respective spines 22 such that the electrodes 26 of the neighboring spines 22 are not horizontally aligned and are instead staggered in comparison to each other. For example, electrodes 26A of spine 22A are not horizontally aligned with electrodes 26B of spine 22B. The end effector 28 can further include a central position sensor 342 attached to a central delivery shaft 340, the central deliver shaft 340 positioned within the center of the end effector 28 and emerging from where the spines 22 of the plurality of spines 22 converge proximally. In other examples, the central position sensor 342 can be disposed on the stem 96.

As shown, each spine 22 of the plurality of spines 22 can include a biased section 320 that extends radially outward from the radius of curvature 328 defined by a remainder of each spine 22. This bias section 320 can cause the electrodes 26 to contact target tissue prior to the remainder of each spine 22 of the plurality of spines 22 contacting the tissue. In other words, the biased sections 320 can be configured to cause the electrodes 26 to be disposed further away from the longitudinal axis 86 such that the electrodes 26 are likely to come in contact with tissue before other portions of the end effector 28. As will be appreciated, this can help to ensure the electrodes 26 make sufficient contact with the tissue.

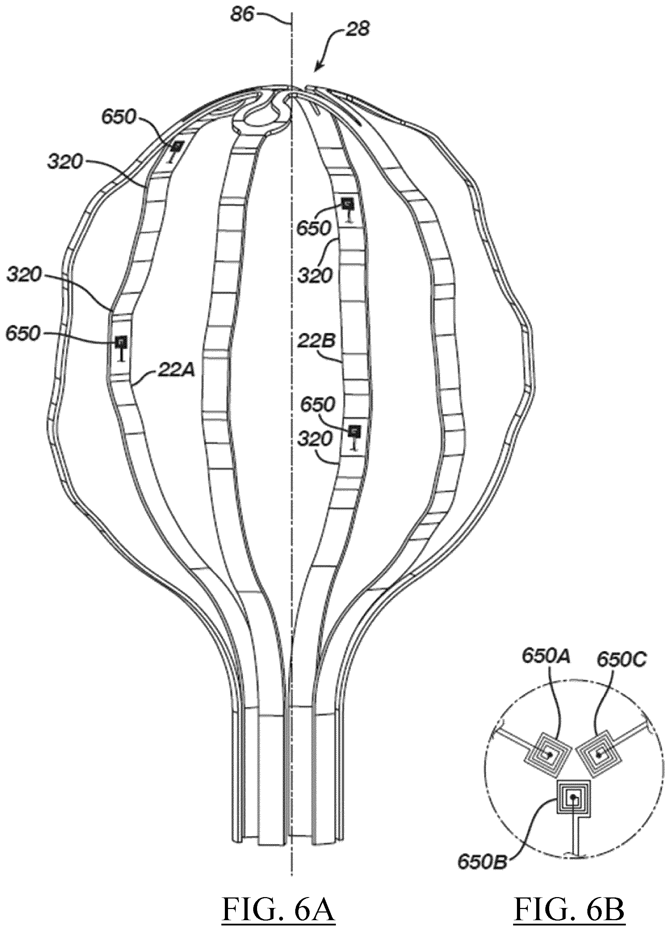

In some examples, as will be shown in further detail in FIGS. 6A-6B, the spines 22 can further include a position sensor 650 disposed at each bias section 320 of each spine 22. The location sensor 650, for example, can be disposed underneath or on either side of a respective electrode 26 as shown in FIG. 6A. As stated above, the end effector 28 can include the central position sensor 342 centrally disposed within the end effector 28. This can be used in conjunction with the location sensors 650 disposed at the biased sections 320 to determine the amount of deflection experienced by the spines 22. This information can be used to determine a magnitude and direction of a force applied to the end effector 28. For example, if a spring constant of the biased sections 320 is known, that information can be used along with the detected locations of electrodes 26A,26B disposed on the biased sections 320 in relation to each other and to the central position sensor 342 to determine the magnitude and direction of force experienced by the electrodes 26. As a result, a detailed image of both the force magnitude and direction exerted onto the end effector 28 can be calculated.

To illustrate, if the basket assembly 28 is brought into contact with tissue on a first side, the electrodes 26 on that first side will be pushed inwardly toward the central position sensor 342 and the electrodes 26 on a second, opposite side will be pushed outward away from the central position sensor 342. By knowing the position of the electrodes 26 in relation to the central position sensor 342 in addition to the spring constant of the spines 22, the magnitude and direction of the force applied to the basket assembly 28 can be determined. As well, the coils disposed on the electrode can be used to determine the location of the electrode in free space as triangulated via external magnetic fields generators.

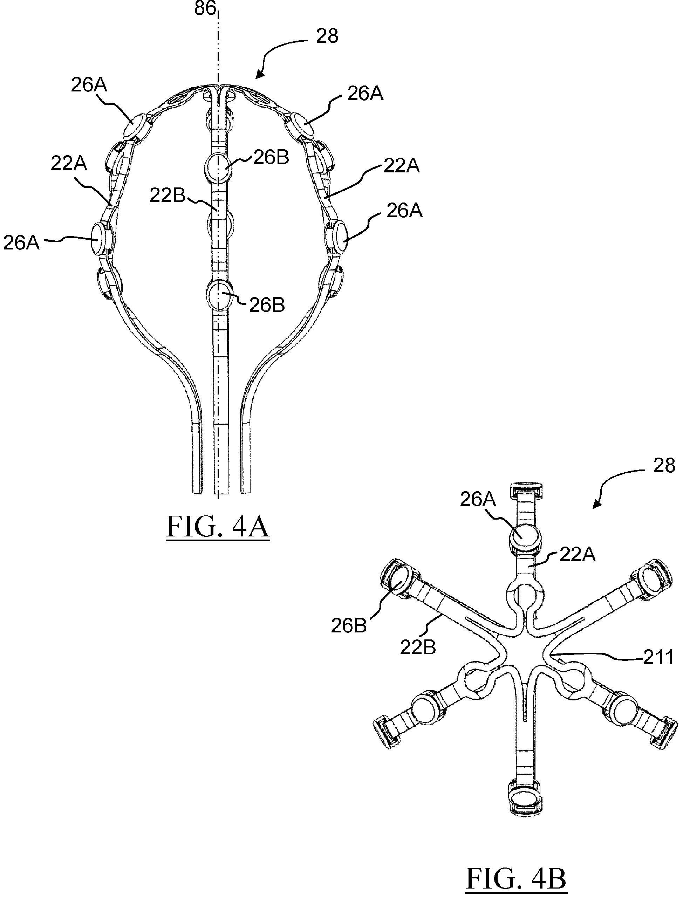

Now referencing FIGS. 4A-4B which illustrate an alternative end effector 28 comprising a plurality of spines 22 that define a basket shape in an expanded configuration, but not including a central position sensor 342. As stated above, each spine 22 of the plurality of spines 22 can extend along a longitudinal axis 86 and can be configured to bow radially outward and away from the longitudinal axis 86 when the end effector 28 is in the expanded configuration. Each spine 22 of the plurality of spines 22 can be configured to converge at a central intersection 211 defining a distal end of the end effector 28. Further, the plurality of spines 22 can include a plurality of electrodes 26 disposed along the spines at biased sections 320 of the spines 22.

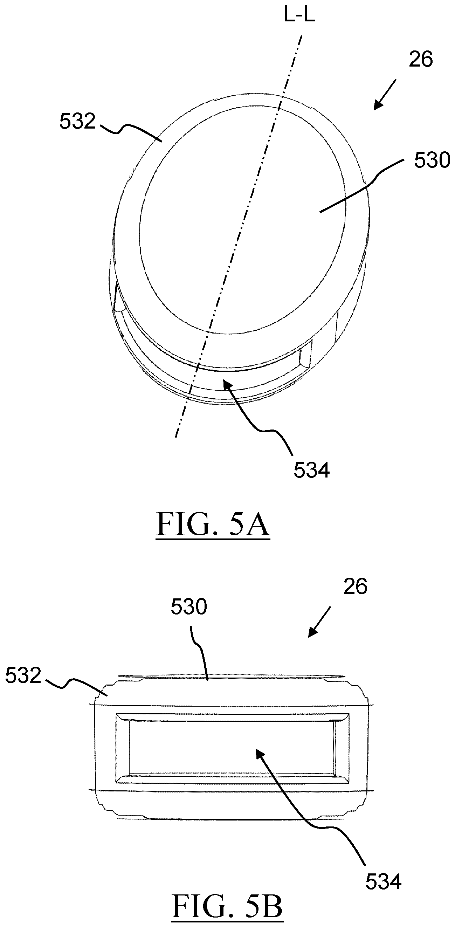

Turning now to FIGS. 5A-5B which illustrate an electrode 26 configured to be disposed along spines 22 of a basket catheter. The electrode 26 can comprise an electrode body 532 that defines a lumen 534 that can be configured to receive the spine 22. In other words, the electrodes 26 can be configured to slide onto a spine 22. The electrode 26 can further include an electrode surface 530 configured to contact target tissue in a patient 23 organ 12. In some examples, the electrode body 532 can comprise an insulated material while the electrode surface 530 is non-insulated in order to ensure ablative energy is delivered only through the electrode surface 530 and to shield the electrode body 532 from the ablative energy. In other examples, the entire electrode 26 can be conductive.

FIGS. 6A illustrates an alternative end effector 28 in an expanded configuration. The end effector 28 can include a plurality of spines 22 extending along a longitudinal axis 86 and defining a basket shape when the end effector 28 is in the expanded configuration. In some examples, at least some of the spines 22 of the plurality of spines 22 can comprise position sensors 650 disposed along the spines 22. Similar to the end effector 28 shown in FIG. 3, each spine 22 of the plurality of spines 22 can be configured to bow radially outward from the longitudinal axis 86 in order to define a radius of curvature 328 for each spine 22. Each spine can further include a biased section 320 extending radially outward from the radius of curvature 328 defined by a remainder of each spine 22.

As stated above, a plurality of position sensors 650 can be disposed along the spines 22. For example, the position sensors 650 can be disposed on the spines 22 at each biased section 22A,22B of each spine 22 such that the position sensors 650 are position beneath or beside the electrodes 26. In some examples, the end effector 28 can include multiple position sensors 650A-C disposed at each biased section 22A,22B of each spine 22. As shown in FIG. 6B, the plurality of position sensors 650A-C can include at least three position sensors 650A-C positioned approximately 60 degrees from each other to from a triple-axis sensor. As will be appreciated, position data collected by the triple-axis sensor can be used to more accurately determine the location and orientation of each biased section 320 of each spine 22.

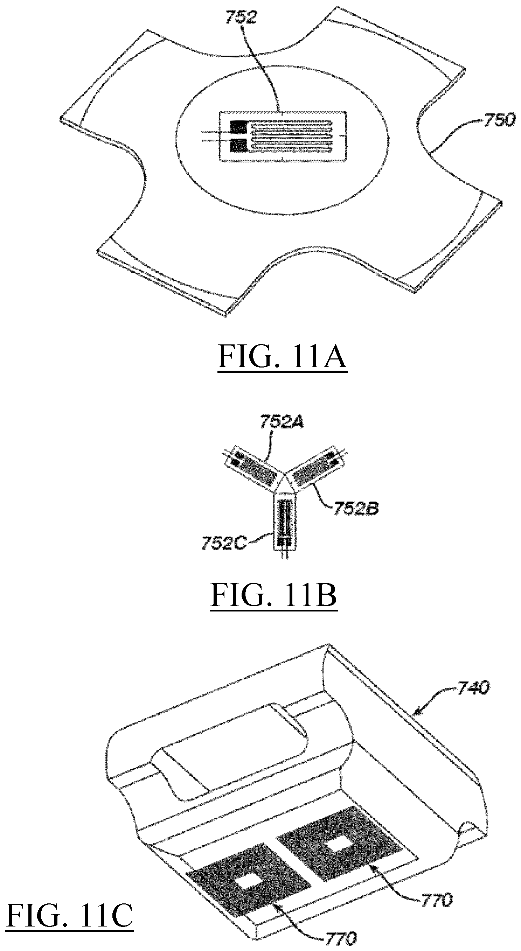

As yet another example, the end effector 28 can alternatively include a strain gauge or a plurality of strain gauges disposed on the biased sections 320 of the spines 22, as will be shown and described in relation to FIG. 11B. The strain gauges can similarly be used to determine the amount of force that the biased sections 320 of the spines 22 experience from detecting a strain applied to the spines 22. In turn, this force data can be used to determine the amount of force applied to each electrode 26.

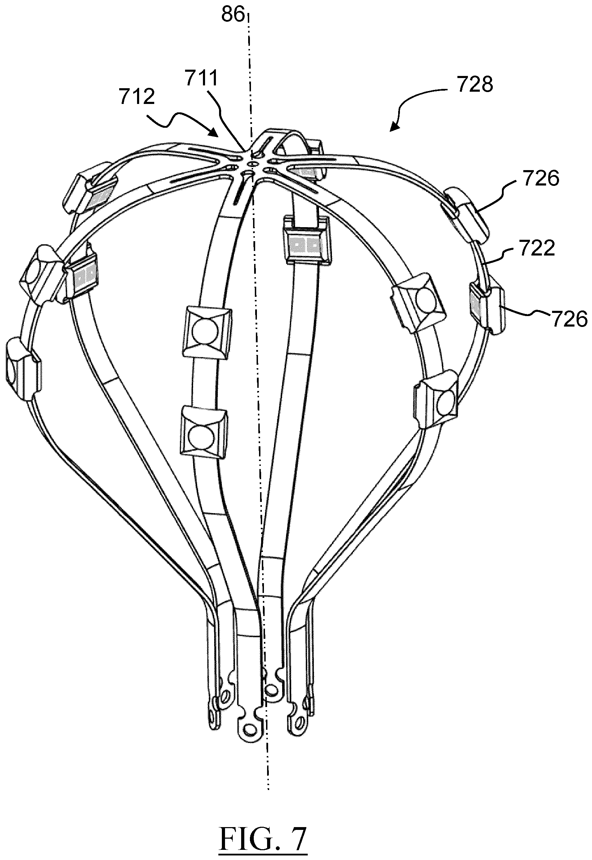

Now referencing FIG. 7 which illustrates an alternative end effector 728 in an expanded configuration. The end effector 728 can include a plurality of spines 722 extending along a longitudinal axis 86 and defining a basket shape when the end effector 728 is in the expanded configuration. The spines 722 can be configured to bow radially outward from a longitudinal axis 86 of the end effector 728. Further, the spines 722 can be configured to converge at a central intersection 711 and define a distal end 712 of the end effector 728. Each spine 722 can include one or more electrode assemblies 726 disposed along the spines 722. As before, each electrode assembly 726 electrode assemblies 726 can be positioned along the spines 726 at a biased section 722 of each spine 722. Alternatively, the spines 22 may not include a biased section but the electrode assemblies 726 can be configured such that the outer-facing portion (the portion of the electrode assembly 726 facing away from the longitudinal axis 86) can be a larger than an inwardly-facing portion (the portion of the electrode assembly 726 facing toward the longitudinal axis 86) Each electrode assembly 726 electrode assemblies 726can further include electromagnetic coils 770 disposed on an inwardly-facing side of the electrode body 740, as will be explained in further detail in FIG. 11C.

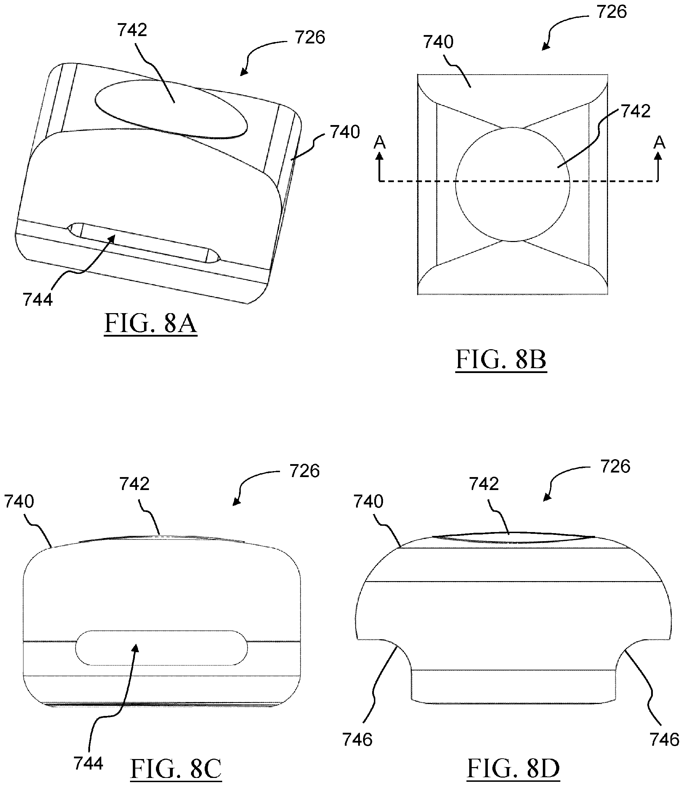

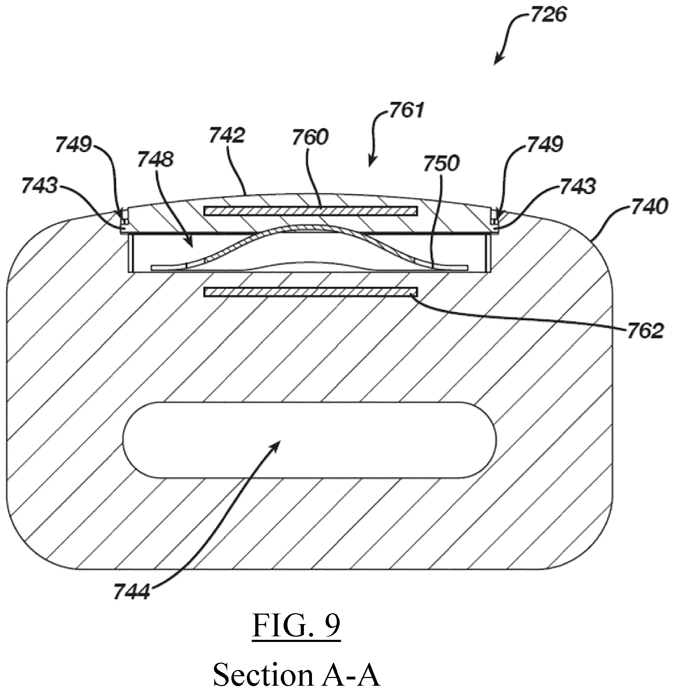

Turning now to FIGS. 8A-8D which illustrate the electrode assemblies 726 disposed along the spines 722 illustrated in FIG. 7. The electrode assembly 726 can include an electrode body 740, an electrode cap 742, and a lumen 744 extending through the electrode body 740 and configured to receive a spine 722. The electrode cap 742 can be positioned centrally on a top-most face of the electrode body 740 as shown in FIG. 8B. The electrode body 740 can define cutouts 746 at respective ends of the lumen 744 as shown in FIG. 8C. This allows the electrode assembly 726 to fully receive the spines 722 and allowed the spines 722 to bend. In some examples, as well as being disposed on a basket catheter as shown in FIG. 7, the electrodes illustrated in FIGS. 8A-8D can similarly be disposed on end effectors 28 comprising biased sections 320, similar to those illustrated in FIGS. 3-4B. FIGS. 9 through 11C further illustrate the details of these electrode assemblies 726.

FIGS. 9-10 illustrate an example electrode assembly 726 similar to the electrode assembly 726 illustrated in FIGS. 8A-8D. As can be seen in FIG. 9, which is a section view of the electrode assembly 726 taken along line A-A shown in FIG. 8B, the electrode assembly 726 can include an electrode body 740 and an electrode cap 742 disposed within a recess 748 defined by the electrode body 740. As previously described, the electrode body 740 can include a lumen 744 extending through the electrode body 740 and configured to receive a spine 722 of an end effector 728. The recess 748 of the electrode body 740 can include a spring member 750 disposed in the recess 748 between the electrode body 740 and the electrode cap 742. The electrode assembly 726 can further include sensor assembly 761. Sensor assembly 761 has a movable coil 760, a resilient member 750 coupled to the first coil 760 and a fixed coil 762. The fixed coil 762 can be connected to the Biosense Webster Carto3 system to transmit a magnetic field at a predetermined frequency and the movable coil 760 can be configured to receive the transmitted magnetic field which induces a current in the movable coil 760. The induced current in movable coil 760 can be used by Carto3 to determine the location of the movable coil or sensor 760 relative to the fixed coil or sensor 762. At least one position sensor or coil 760 can be disposed within the electrode cap 742 and at least one position sensor or coil 762 can be disposed within the electrode body 740 between the lumen 744 and the recess 748.

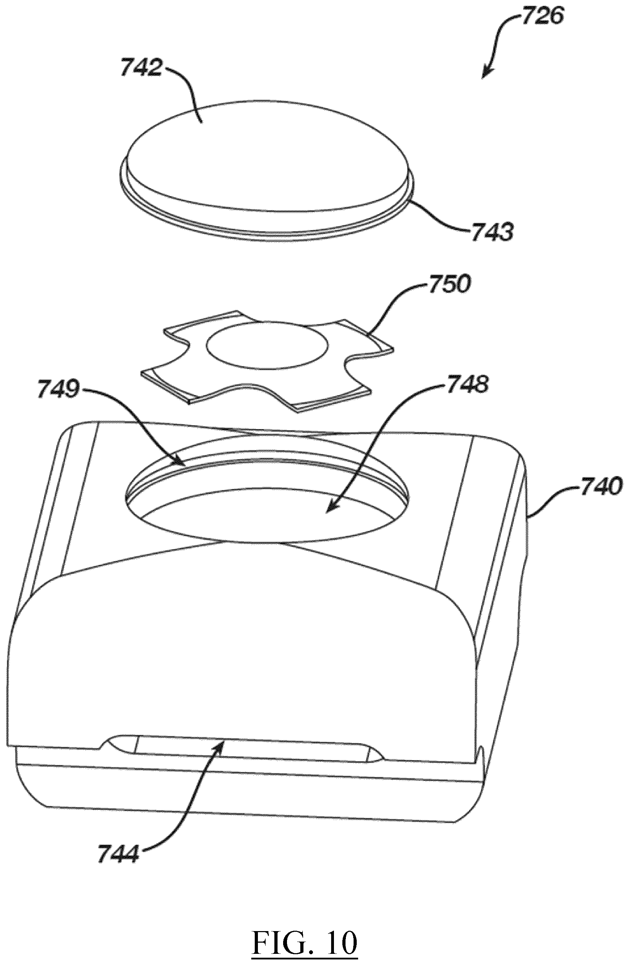

In some examples, the spring member 750 can be configured to apply a force to the electrode cap 742 that can cause the electrode cap 740 to extend outwardly and away from the electrode body 740. As shown in FIG. 10, the recess 748 of the electrode body 740 can include a lip 749 extending around the recess 748 and the electrode cap 740 can include an edge 743 extending around a circumference of the electrode cap 742. The lip 749 of the recess 748 can be configured to help retain the electrode cap 740 on the electrode body 740. For example, the edge 743 of the electrode cap 740 can be configured to slide along the lip 749 but not be removed from the lip 749 to prevent the removal of the electrode cap 740. The spring member 750 and the position sensors 760,762 can be used together to calculate the amount of force applied to the electrode assemblies 726, for example, when the electrode assemblies 726 are brought into contact with tissue. For example, if the spring constant is known for the spring member 750, the disclosed technology can determine the force applied to the electrode cap 742 by determining the displacement of the position sensor 760 in the electrode cap 742 in relation to the position sensor 762 in the electrode body 740 and correlating the displacement to the spring constant of the spring member 750. In this way, the amount of force applied to each electrode assembly 726 can be determined and output for view by a physician.

Now referencing FIG. 11A which illustrates the spring member 750 that can be disposed within the electrode assembly 726 illustrated in FIGS. 7-10. In some examples, the spring member 750 can alternatively or in addition include a strain gauge 752 disposed on the spring member 750. The strain gauge 752 can be configured to determine the amount of force applied by spring member 750 onto the electrode cap 742. In some examples, the spring member 750 can further include three strain gauges 752A-C disposed on the spring member 750 and separated approximately 60 degrees from each other, as shown in FIG. 11B for determining the magnitude and direction in which the force applied to the electrode cap 742. In some examples, the strain gauges 752A-C can include flexible circuits configured to detect a strain when flexed.

FIG. 11C illustrates an underside view of the electrode body 740. In this example the electrode body 740 can include one or more electromagnetic coils 770 disposed on an underside of the electrode body 740. In some examples, the electromagnetic coils 770 of the electrode body 740 can include a first and second electromagnetic coil 770 that can comprise a dual axis sensor. In other examples, the electrode body 740 can include only one electromagnetic coil or more than two electromagnetic coils, or any number as suitable for the particular application. Each electromagnetic coil 770 can be configured to, when subjected to an electromagnetic field, output an electrical current and detect a position of the electrode assembly 726. As will be appreciated, by including one or more electromagnetic coils 770 on the electrode assembly 726, the disclosed technology can be configured to determine the position and orientation of each electrode assembly 726 on the basket catheter 728.

The disclosed technology described herein can be further understood according to the following clauses:
Clause 1: An end effector for a medical device comprising: a plurality of spines extending along a longitudinal axis to define a basket assembly, the plurality of spines configured to bow radially outward from the longitudinal axis to define a radius of curvature with respect to the longitudinal axis and to transition between an expanded configuration and a collapsed configuration, each spine of the plurality of spines including a section extending radially outward from the radius of curvature defined by a remainder of each spine; and at least one electrode disposed on the spring bias section for each to the plurality of spines.
Clause 2: The end effector of clause 1, a plurality of electrodes being disposed on each spine of the plurality of spines.
Clause 3: The end effector of clause 1, each electrode of a plurality of electrodes being disposed on a respective section on each spine of the plurality of spines.
Clause 4: The end effector of clause 3, the sections of each spine of the plurality of spines configured to cause each respective electrode of the plurality of electrodes to contact tissue prior to the remainder of each spine contacting the tissue when the end effector is brought into contact with tissue.
Clause 5: The end effector of clause 1, the at least one electrode being configured to deliver ablative energy to tissue.
Clause 6: The end effector of clause 3, further comprising a location sensor disposed on each respective section and configured to detect a position of the section.
Clause 7: The end effector of clause 6, each location sensor disposed on the section underneath a respective electrode of the plurality of electrodes.
Clause 8: The end effector of clause 1, each electrode comprising: an electrode body defining a lumen extending therethrough and a recess; an electrode cap disposed at least partially in the recess; and a spring member disposed in the recess between the electrode body and the electrode cap, the spring member configured to cause the electrode cap to extend outwardly from the electrode body.
Clause 9: The end effector of clause 8, further comprising a sensor coupled to the spring member, the sensor configured to detect a force applied to the electrode.
Clause 10: The end effector of clause 8, the electrode body further defining a lip extending around the recess, the lip configured to prevent the electrode cap from being removed from the recess.
Clause 11: The end effector of clause 8, the lumen configured to receive a spine of the plurality of spines.
Clause 12: The end effector of clause 8 further comprising a position sensor disposed on the electrode body and configured to output a current when subjected to an electromagnetic field to detect a position of the electrode.
Clause 13: The end effector of clause 12, the position sensor being disposed on an inwardly-facing side of the electrode body that faces toward the longitudinal axis.
Clause 14: The end effector of clause 12, the position sensor being a first position sensor, the end effector further comprising a second position sensor disposed on the electrode body and configured to output a current when subjected to an electromagnetic field to detect a position of the electrode.
Clause 15: The end effector of clause 14, the first position sensor and the second position sensor comprising a dual axis sensor.
Clause 16: An electrode assembly comprising: an electrode body extending along a longitudinal axis and defining a recess; an electrode cap disposed at least partially in the recess; and a spring member disposed in the recess between the electrode body and the electrode cap, the spring member configured to cause the electrode cap to extend outwardly from the electrode body.
Clause 17: The electrode assembly of clause 16, further comprising a sensor coupled on the spring member, the sensor configured to detect a force applied to the electrode.
Clause 18: The electrode assembly of clause 16, the electrode body further defining a lip extending around the recess, the lip configured to prevent the electrode cap from being removed from the recess.
Clause 19: The electrode assembly of clause 16, the electrode body further defining a lumen configured to receive a spine, the spine configured to support the electrode assembly.
Clause 20: The electrode assembly of clause 16 further comprising a position sensor disposed on the electrode body and configured to output a current when subjected to an electromagnetic field to detect a position of the electrode.
Clause 21: The electrode assembly of clause 20, the position sensor being disposed on an inwardly-facing side of the electrode body.
Clause 22: The electrode assembly of clause 20, the position sensor being a first position sensor, the electrode assembly further comprising a second position sensor disposed on the electrode body and configured to output a current when subjected to an electromagnetic field to detect a position of the electrode.
Clause 23: The electrode assembly of clause 22, the first position sensor and the second position sensor comprising a dual axis sensor.
Clause 24: The electrode assembly of clause 16, further comprising a first position sensor disposed on the electrode cap and a second position sensor disposed on the electrode body.

The embodiments described above are cited by way of example, and the present invention is not limited by what has been particularly shown and described hereinabove. Rather, the scope of the invention includes both combinations and sub combinations of the various features described hereinabove, as well as variations and modifications thereof which would occur to persons skilled in the art upon reading the foregoing description and which are not disclosed in the prior art.

## Claims

1. An end effector for a medical device comprising:
a plurality of spines extending along a longitudinal axis to define a basket assembly, the plurality of spines configured to bow radially outward from the longitudinal axis to define a radius of curvature with respect to the longitudinal axis and to transition between an expanded configuration and a collapsed configuration, each spine of the plurality of spines including a section extending radially outward from the radius of curvature defined by a remainder of each spine; and
at least one electrode disposed on the section for each spine of the plurality of spines.

2. The end effector of claim 1, a plurality of electrodes being disposed on each spine of the plurality of spines.

3. The end effector of claim 1, each electrode of a plurality of electrodes being disposed on a respective section on each spine of the plurality of spines.

4. The end effector of claim 3, the sections of each spine of the plurality of spines configured to cause each respective electrode of the plurality of electrodes to contact tissue prior to the remainder of each spine contacting the tissue when the end effector is brought into contact with tissue.

5. The end effector of claim 1, the at least one electrode being configured to deliver ablative energy to tissue.

6. The end effector of claim 3, further comprising a location sensor disposed on each respective section and configured to detect a position of the section, optionally each location sensor disposed on the section underneath a respective electrode of the plurality of electrodes.

7. The end effector of claim 1, each electrode comprising:
an electrode body defining a lumen extending therethrough and a recess;
an electrode cap disposed at least partially in the recess; and
a spring member disposed in the recess between the electrode body and the electrode cap, the spring member configured to cause the electrode cap to extend outwardly from the electrode body.

8. The end effector of claim 7, further comprising a force sensor assembly including a first coil coupled to the spring member, and a second coil fixed to the electrode, the first coil sensor being configured to output a current in response to a magnetic field for a determination of a force applied to the electrode.

9. The end effector of claim 7, the electrode body further defining a lip extending around the recess, the lip configured to prevent the electrode cap from being removed from the recess.

10. The end effector of claim 7, the lumen configured to receive a spine of the plurality of spines.

11. The end effector of claim 7 further comprising a position sensor disposed on the electrode body and configured to output a current when subjected to an electromagnetic field to detect a position of the electrode.

12. The end effector of claim 11, the position sensor being disposed on an inwardly-facing side of the electrode body that faces toward the longitudinal axis.

13. The end effector of claim 11, the position sensor being a first position sensor, the end effector further comprising a second position sensor disposed on the electrode body and configured to output a current when subjected to an electromagnetic field to detect a position of the electrode, optionally the first position sensor and the second position sensor comprising a dual axis sensor.

14. An electrode assembly comprising:
an electrode body extending along a longitudinal axis and defining a recess;
an electrode cap disposed at least partially in the recess; and
a spring member disposed in the recess between the electrode body and the electrode cap, the spring member configured to cause the electrode cap to extend outwardly from the electrode body.

15. The electrode assembly of claim 14, further comprising i) a force sensor assembly including a first coil coupled to the spring member and a second coil fixed to the electrode body, the force sensor assembly configured to detect a force applied to the electrode, or ii) comprising a position sensor disposed on the electrode body and configured to output a current when subjected to an electromagnetic field to detect a position of the electrode.

16. The electrode assembly of claim 14, the electrode body further defining i) a lip extending around the recess, the lip configured to prevent the electrode cap from being removed from the recess, or ii) a lumen configured to receive a spine, the spine configured to support the electrode assembly.
